(19) Europäisches Patentamt
European Patent Office
Office européen des brevets

(11) Publication number: **0 048 548**
**B1**

(12) # EUROPEAN PATENT SPECIFICATION

(45) Date of publication of patent specification: **14.11.84**

(51) Int. Cl.$^3$: **A 61 K 31/41** // C07D249/08, C07D405/06, A01N43/64

(21) Application number: **81303752.0**

(22) Date of filing: **18.08.81**

(54) **Pharmaceutical compositions of fungicidal triazole derivatives.**

(30) Priority: **28.08.80 GB 8027800**

(43) Date of publication of application:
**31.03.82 Bulletin 82/13**

(45) Publication of the grant of the patent:
**14.11.84 Bulletin 84/46**

(84) Designated Contracting States:
**BE CH DE FR GB IT LI LU NL SE**

(56) References cited:
**EP-A-0 011 768**
**EP-A-0 011 769**
**EP-A-0 015 756**
**EP-A-0 017 080**
**EP-A-0 036 153**
**EP-A-0 047 594**
**DE-A-2 623 139**

(73) Proprietor: **IMPERIAL CHEMICAL INDUSTRIES PLC**
**Imperial Chemical House Millbank**
**London SW1P 3JF (GB)**

(72) Inventor: **Parry, Keith Peter**
**10 Calder Court**
**Maidenhead Berkshire (GB)**
Inventor: **Worthington, Paul Anthony**
**4 Oakhurst Road Maidenhead Court Park**
**Maidenhead Berkshire (GB)**

(74) Representative: **Atkinson, John David et al**
**Imperial Chemical Industries PLC Legal**
**Department : Patents PO Box 6 Bessemer Road**
**Welwyn garden City Herts, AL7 1HD (GB)**

**EP 0 048 548 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to pharmaceutical and veterinary compositions of fungicidal triazole derivatives, and in particular it relates to such compositions which are orally or topically active against fungus diseases of humans and other animals. The compositions of the invention are especially useful for treatment of candidiosis and human dermatophyte infections.

Our co-pending European Patent Application EP—A—0015756 discloses a novel class of triazole derivatives of the general formula:—

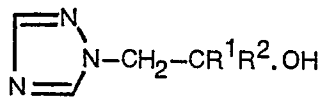

I

wherein $R^1$ is alkyl, cycloalkyl or optionally substituted phenyl and $R^2$ is optionally substituted phenyl or optionally substituted benzyl, and the acid addition salts and metal complexes thereof, and chemical processes for the manufacture of these compounds. The said patent application also discloses that these compounds and compositions containing them possess fungicidal activity, but the only such fungicidal activity which is exemplified therein is activity against fungus disease of plants.

Similar compounds wherein $R^1$ is phenyl substituted by an optionally-substituted phenyl or cycloalkyl group and $R^2$ is an optionally-substituted phenyl or benzyl radical, are disclosed in prior European Patent Applications Numbers 11768 and 11769.

The present invention therefore provides a pharmaceutical or veterinary composition for use as an antifungal in human or other animals which comprises a triazole derivative of the formula II

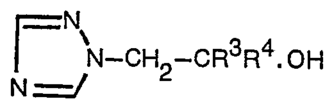

II

wherein $R^3$ is a 1—6C alkyl radical, a 3—7C cycloalkyl radical, a phenyl radical, a phenyl radical which is substituted by one or more substituents selected from halogen atoms and 1—5C alkyl, 1—4C alkoxy, 1—4C haloalkyl, 1—4C haloalkoxy, nitro, phenoxy, benzyl, benzyloxy, halobenzyloxy, 1—4C alkylenedioxy, 1—4C haloalkylenedioxy, amino, 1—4C alkylamino, di(1—4C alkyl)amino, hydroxy, morpholino, carboxy and 2—5C alkoxycarbonyl radicals; and $R^4$ is a phenyl, benzyl or $\alpha$-methylbenzyl radical, or a phenyl, benzyl or $\alpha$-methylbenzyl radical each substituted as defined above, or an acid addition salt thereof, but excluding compounds wherein $R^3$ is a 2,4 dichlorophenyl radical and $R^4$ is a phenyl or 2-, 3- or 4-chlorophenyl radical, or $R^3$ is a phenyl or 4-chlorophenyl radical and $R^4$ is a 2- or 4-chlorophenyl radical, together with a pharmaceutically or veterinarily acceptable diluent or carrier.

The compounds of the formula I contain a chiral centre when $R^3$ and $R^4$ are different. Such compounds are generally obtained in the form of racemic mixtures, but these can be separated into the individual isomers by methods known in the art.

The alkyl radical $R^3$ can be a straight or branched chain group, for example methyl, ethyl, propyl, (*n*- or *iso*-propyl) and butyl (*n*-, *sec*- or *t*-butyl) radicals, and preferred such alkyl radicals are those containing 1 to 4 carbon atoms.

A suitable value for $R^1$ when it is a cycloalkyl radical is, for example, a cyclopentyl or cyclohexyl radical.

Examples of suitable substituents in the phenyl radical, $R^3$ and $R^4$, or in the phenyl moiety of the benzyl radical, $R^4$, are fluorine, chlorine or bromine atoms, methyl, ethyl, propyl (*n*- or *iso*-propyl), butyl (*n*-, *sec*-, *iso*- or *t*-butyl), methoxy, ethoxy, trifluoromethyl, 1,1,2,2-tetrafluoroethyl, trifluoromethoxy, 1,1,2,2-tetrafluoroethoxy, chlorobenzyloxy, fluorobenzyloxy, methylenedioxy, 1,2-ethylenedioxy, 1,2-propylenedioxy, difluoromethylenedioxy, nitro, amino, methylamino, dimethylamino, hydroxy, morpholino, methoxycarbonyl and ethoxycarbonyl radicals. The alkyl moiety of the benzyl can be substituted with methyl so that $R^4$ is a 1-phenylethyl radical. Suitably the phenyl and benzyl are unsubstituted or substituted with 1, 2 or 3 ring substituents as defined above. Examples of these groups from which $R^3$ may be selected are phenyl, 2-, 3- or 4- chlorophenyl, 2,3- or 2,6-dichlorophenyl, 2-, 3- or 4-fluorophenyl, 2,3- or 2,6-difluorophenyl, 2-, 3- or 4-bromophenyl, 2-chloro-4-fluorophenyl, 2-fluoro-4-chlorophenyl, 2-chloro-6-fluorophenyl, 2-, 3- or 4-methoxyphenyl, 2,4-dimethoxyphenyl, 2-, 3- or 4-ethoxyphenyl, 2-, 3- or 4-nitrophenyl, 2-chloro-4-nitrophenyl, 2-chloro-5-nitrophenyl, 2-, 3- or 4-methylphenyl, 2,4-dimethylphenyl, 2-, 3- or 4-t-butylphenyl, 2-, 3- or 4-trifluoromethylphenyl, 2-, 3- or 4-trifluoromethoxyphenyl, 2-, 3- or 4-(1,1,2,2-tetrafluoroethyl)phenyl, 2,3-(difluoromethylenedioxy)phenyl, 2-fluoro-4-methoxyphenyl, 2-methoxy-4-fluorophenyl, 2-methoxy-4-chlorophenyl, 2-, 3- or 4-phenoxyphenyl, 2-, 3- or 4-phenylphenyl (2-, 3- or 4-biphenylyl), 2-, 3- or 4-benzylphenyl, 2-, 3- or 4-benzyloxyphenyl, 2-, 3- or 4-(4-chloro- or 4-fluorobenzyloxy)phenyl, 2-, 3- or 4-aminophenyl, 2-, 3- or 4-(N,N-dimethylamino)phenyl, 2-, 3- or 4-hydroxyphenyl, 2-, 3- or 4-carboxyphenyl, 2-, 3- or 4-(methoxycarbonyl)phenyl and 2-, 3- or 4-morpholinophenyl and $R^4$ may be selected from the same radicals, benzyl, $\alpha$-methylbenzyl, and the corresponding ring substituted benzyl and $\alpha$-methylbenzyl groups, bearing substituents as defined above in $R^3$.

The salts can be salts with inorganic or organic acids e.g. hydrochloric, nitric, sulphuric, acetic, p-toluenesulphonic or maleic acid.

Examples of compounds of the formula II, which may be used in the composition of the invention are shown in the following Table I.

TABLE I

| Compound No. | $R^3$ | $R^4$ | Melting point (°C.) |
|---|---|---|---|
| 1 | $C_6H_5$— | $C_6H_5CH_2$— | 124—125 |
| 2 | $C_6H_5$— | 4-Cl—$C_6H_4CH_2$— | 144—145 |
| 3 | $C_6H_5$— | 4-F—$C_6H_4CH_2$— | 116—118 |
| 4 | 4-Cl—$C_6H_4$— | 4-Cl—$C_6H_4CH_2$— | 80—83 |
| 5 | 4-Cl—$C_6H_4$— | $C_6H_5CH_2$— | 109—111 |
| 6 | 4-F—$C_6H_4$— | $C_6H_5CH_2$— | 141—142 |
| 7* | $C_6H_5$— | 2,4-$Cl_2$—$C_6H_3CH_2$— | 104—106 |
| 8+ | 4-F—$C_6H_4$— | 4-F—$C_6H_4CH_2$— | 154—156 |
| 9 | 4-F—$C_6H_4$— | 4-Cl—$C_6H_4CH_2$— | 168—170 |
| 10 | t-Bu | $C_6H_5CH_2$— | 110—111 |
| 11 | t-Bu | 4-Cl—$C_6H_4CH_2$— | 86—87 |
| 12 | t-Bu | 4-F—$C_6H_4CH_2$— | 146—148 |
| 13 | $C_6H_5$— | 2-F—$C_6H_4CH_2$— | 133—134 |
| 14 | 4-Cl—$C_6H_4$ | 2-F—$C_6H_4CH_2$— | 95—96 |
| 15 | $C_6H_5$— | 2-Cl—$C_6H_4CH_2$— | 69—71 |
| 16 | 4-MeO—$C_6H_4$— | $C_6H_5CH_2$— | 100—103 |
| 17 | $C_6H_5$— | $C_6H_5$— | 128—129 |
| 18+ | 4-F—$C_6H_4$— | 4-F—$C_6H_4CH_2$— | 161—163 |
| 19 | $C_6H_5$— | 2,4-$Cl_2$—$C_6H_3CH_2$— | 104—106 |
| 20 | t-Bu | 2-Cl—$C_6H_4CH_2$— | 74—75 |
| 21 | t-Bu | 2-F—$C_6H_4CH_2$— | 96—98 |
| 22 | t-Bu | 3-Cl—$C_6H_4CH_2$— | 88—89 |
| 23 | t-Bu | 3-$CF_3$—$C_6H_4CH_2$— | 106—107 |
| 24 | $C_6H_5$— | 4-t-Bu—$C_6H_4CH_2$— | 80—83 |
| 25 | 4-Cl—$C_6H_4$— | 4-F—$C_6H_4$— | 154—155 |
| 26 | 4-F—$C_6H_4$— | 4-F—$C_6H_4$— | 170—171 |
| 27 | 4-F—$C_6H_4$— | $C_6H_5$— | 139—140 |

TABLE I (Cont'd)

| Compound No. | $R^3$ | $R^4$ | Melting point (°C.) |
|---|---|---|---|
| 28 | i-Bu | $C_6H_5$— | 94—95 |
| 29 | n-Bu | 4-Cl—$C_6H_5$— | 95—97 |
| 30 | t-Bu | 2-Cl-4-F—$C_6H_3CH_2$— | 95 |
| 31 | t-Bu | 2-F-4-Cl—$C_6H_3CH_2$— | 104—106 |
| 32 | $C_6H_5$— | 4-t-Bu—$C_6H_4$— | 131—135 |
| 33 | 4-Cl—$C_6H_4$— | 2-F—$C_6H_4$— | 144—145 |
| 34 | 4-F—$C_6H_4$— | 2-Cl—$C_6H_4$— | 115—116 |
| 35 | 4-F—$C_6H_4$— | 2-F—$C_6H_4$— | 120—123 |
| 36 | 4-Cl—$C_6H_4$— | 2-Me—$C_6H_4$— | 157—158 |
| 37 | 2,4-$Cl_2$—$C_6H_3$— | 4-F—$C_6H_4$— | 137—138 |
| 38 | 2-Cl—$C_6H_4$— | 4-MeO—$C_6H_4$— | 184—185 |
| 39 | 2,3-diCl—$C_6H_3$— | 2-Cl—$C_6H_4$— | 149—151 |
| 40 | 2,3-diCl—$C_6H_3$— | 2-Fl$C_6H_4$— | 146—147 |
| 41 | 4-$C_6H_5CH_2O$—$C_6H_4$— | $C_6H_5$— | 134—136 |
| 42 | 4-(4-Cl—$C_6H_4CH_2O$)—$C_6H_4$— | $C_6H_5$— | 98—100 |
| 43 | 3-Cl—$C_6H_4$— | 4-Cl—$C_6H_4$— | 139—142 |
| 44 | 4-(4-F—$C_6H_4CH_2O$)—$C_6H_4$— | $C_6H_5$— | 105—107 |
| 45 | 3-Cl—$C_6H_4$— | 4-F—$C_6H_4$— | 190—193 |
| 46 | 3-Cl—$C_6H_4$— | 4-MeS—$C_6H_4$— | 58—60 |
| 47 | 2-Me—$C_6H_4$— | 4-F—$C_6H_4$— | 200—201 |
| 48 | 2-Me—$C_6H_4$— | 4-Cl—$C_6H_4$— | 157—158 |
| 49 | t-Bu | 4-Br—$C_6H_4CH_2$— | 111—115 |
| 50 | t-Bu | 2,4-$F_2$—$C_6H_3CH_2$— | 140 |
| 51 | t-Bu | 2-MeO—$C_6H_4CH_2$— | 113—116 |
| 52 | $C_6H_5$— | 2-F—$C_6H_4$— | 126—128 |
| 53 | $C_6H_5$— | 2-Me—$C_6H_4$— | 161—162 |
| 54 | 4-F—$C_6H_4$— | 4-$MeO_2C$—$C_6H_4$— | 164—166** |
| 55 | 4-EtO—$C_6H_4$— | 4-Cl—$C_6H_4$— | 126—127 |
| 56 | 4-$CF_2H$ . O—$C_6H_4$— | 4-Cl—$C_6H_4$— | glass |
| 57 | 4-$CF_2H$ . O—$C_6H_4$— | 2-Cl—$C_6H_4$— | glass |

TABLE I (Cont'd)

| Compound No. | $R^3$ | $R^4$ | Melting point (°C.) |
|---|---|---|---|
| 58 | 2-Br—$C_6H_4$— | 4-Cl—$C_6H_4$— | 151—152 |
| 59 | 2-Br—$C_6H_4$— | 4-F—$C_6H_4$— | 109—111 |
| 60 | 4-$NO_2$—$C_6H_4$— | $C_6H_5$ | 164—166 |
| 61 | 2-Cl-5-$NO_2$—$C_6H_3$— | $C_6H_5$ | 206—208 |
| 62 | 4-Me—$C_6H_4$— | $C_6H_5$ | 125—127 |
| 63 | 2-MeO—$C_6H_4$— | $C_6H_5$ | 113—116 |
| 64 | 2-MeO—$C_6H_4$— | 4-Cl—$C_6H_4$— | 129—131 |
| 65 | 2-MeO-$C_6H_4$— | 4-F-$C_6H_4$— | 133—135 |
| 66 | 4-Br—$C_6H_4$— | $C_6H_5$— | 104—105 |
| 67 | 4-Br—$C_6H_4$— | 4-Br—$C_6H_4$— | 159—160 |
| 68 | 4-Br—$C_6H_4$— | 4-Cl—$C_6H_4$— | 138—139 |
| 69 | 2-Me—$C_6H_4$— | 4-Me—$C_6H_4$— | 117—120 |
| 70 | 2-Cl—$C_6H_4$— | 4-Me—$C_6H_4$— | 169—170 |
| 71 | 2-F—$C_6H_4$— | 4-Me—$C_6H_4$— | 156—158 |
| 72 | 4-F—$C_6H_4$— | 4-$NO_2$—$C_6H_4$— | 145—148 |
| 73 | 4-Me—$C_6H_4$— | 4-Me—$C_6H_4$— | 153—154** |
| 74 | 3-$NO_2$—$C_6H_4$— | $C_6H_5$— | 212—215** |
| 75 | 2-Cl—$C_6H_4$— | 2-Cl—$C_6H_4$— | 164—166 |
| 76 | 3-$CF_3$—$C_6H_4$— | $C_6H_5$— | 115—117 |
| 77 | 4-$CF_4$—$C_6H_4$— | $C_6H_5$— | 207—210** |
| 78 | 3-$CF_3$—$C_6H_4$— | 4-Cl—$C_6H_4$— | 104—106 |
| 79 | 4-$CF_3O$—$C_6H_4$— | $C_6H_5$ | glass |
| 80 | 4-$CF_3O$—$C_6H_4$— | 4-Cl—$C_6H_4$— | 107—108 |
| 81 | 4-$CF_3O$—$C_9H_4$— | 2-Cl—$C_6H_4$— | 99—100 |
| 82 | 2-$CF_2H$ . $CF_2O$—$C_6H_4$— | $C_6H_5$ | 188—189 |
| 83 | 2-$CF_2H$ . $CF_2O$—$C_6H_4$— | 4-Cl—$C_6H_4$— | 94—95 |
| 84 | 4-$CF_2H$ . $CF_2O$—$C_6H_4$— | $C_6H_5$ | glass |
| 85 | 4-$CF_2H$ . $CF_2O$—$C_6H_4$— | 4-$CF_2H$ . $CF_2O$—$C_6H_4$— | glass |
| 86 | 4-$CF_2H$ . $CF_2O$—$C_6H_4$— | 4-Cl—$C_6H_4$— | glass |
| 87 | Et | $C_6H_5$ | 96—97 |

TABLE I (Cont'd)

| Compound No. | $R^3$ | $R^4$ | Melting point (°C.) |
|---|---|---|---|
| 88 | Et | 4-Cl—$C_6H_4$— | 105—106 |
| 89 | Et | 4-F—$C_6H_4$— | 94 |
| 90 | n-Pr | $C_6H_5$ | 77—79 |
| 91 | cyclo-Pr | $C_6H_5$ | 91—92 |
| 92 | cyclo-Pr | 4-Cl—$C_6H_4$— | oil |
| 93 | cyclo-Pr | 4-MeO—$C_6H_4$— | 101—102 |
| 94 | n-Bu | $C_6H_5$— | 62—63 |
| 95 | n-Bu | 4-F—$C_6H_4$— | 93—95 |
| 96 | n-Bu | 2,4-$Cl_2$—$C_6H_3$— | 106—108 |
| 97 | t-Bu | $C_6H_5$— | 75 |
| 98 | t-Bu | 4-Cl—$C_6H_4$— | 70—73 |
| 99 | t-Bu | 4-F—$C_6H_4$— | 92—93 |
| 100 | t-Bu | 4-MeO—$C_6H_4$— | 48 |
| 101 | n-$C_6H_{13}$— | 4-F—$C_6H_4$— | 97—99 |
| 102 | cyclohexyl | $C_6H_5$— | 110—111 |
| 103 | cyclohexyl | 4-Cl—$C_6H_4$— | 45 |
| 104 | cyclohexyl | 4-MeO—$C_6H_4$— | 92—94 |
| 105 | t-Bu | 4-MeO—$C_6H_4$ . $CH_2$— | 130—131 |
| 106 | 4-Cl-3-$NO_2$—$C_6H_3$— | $C_6H_5$— | 139—141 |
| 107 | 3-Cl—$C_6H_4$— | $C_6H_5$— | 108—110 |
| 108 | 3-F—$C_6H_4$— | 4-Cl—$C_6H_4$— | 55—59 |
| 109 | 3-F—$C_6H_4$— | 4-F—$C_6H_4$— | 116—118 |
| 110 | 4-MeO—$C_6H_4$— | $C_6H_5$— | 88—89 |
| 111 | 4-PhO—$C_6H_4$— | $C_6H_5$— | 103—105 |
| 112 | 2-MeO—$C_6H_4$— | 4-MeO—$C_6H_4$— | 133—134 |
| 113 | 2-F—$C_6H_4$— | 4-MeO—$C_6H_4$— | 144—145 |
| 114 | 4-$NO_2$—$C_6H_4$— | 4-Cl—$C_6H_4$— | 157—158 |
| 115 | 4-MeO—$C_6H_4$— | 4-Cl—$C_6H_4$— | 105—106 |
| 116 | 4-PhO—$C_6H_4$— | 2-Cl—$C_6H_4$— | 100—102 |

TABLE I (Cont'd)

| Compound No. | $R^3$ | $R^4$ | Melting point (°C.) |
|---|---|---|---|
| 117 | 4-PhO—$C_6H_4$— | 2-F—$C_6H_4$— | 132—133 |
| 118 | 2,4-$Cl_2$—$C_6H_3$— | 4-MeO—$C_6H_4$— | 178—180 |
| 119 | 4-MeO—$C_6H_4$— | 4-F—$C_6H_4$— | 124—125 |
| 120 | 4-$CF_2H$ . $CF_2O$—$C_6H_4$— | 2,4-$Cl_2C_6H_3$— | 125—126 |
| 121 | 4-$MeO_2C$—$C_6H_4$— | $C_6H_5$— | 70—72 |
| 122 | 4-Me—$C_6H_4$— | 4-Cl—$C_6H_4$— | 134—135 |
| 123 | 4-Me—$C_6H_4$— | 4-F—$C_6H_4$— | 124—125 |
| 124 | 2-MeO—$C_6H_4$— | 2,4-$Cl_2$—$C_6H_3$— | 152—153 |
| 125 | 3-PhO—$C_6H_4$— | 4-Cl—$C_6H_4$— | 177—178** |
| 126 | 4-PhO—$C_6H_4$— | 4-F—$C_6H_4$— | 138—140 |
| 127 | 4-i-PrO—$C_6H_4$— | 4-Cl—$C_6H_4$— | 151—153 |
| 128 | n-Bu | 4-Me—$C_6H_4$— | 80—81 |
| 129 | n-$C_6H_{13}$— | 4-Me—$C_6H_4$— | 76—78 |
| 130 | 4-$CF_3O$—$C_6H_4$— | 2-F—$C_6H_4$— | glass |
| 131 | 4-$CF_3O$—$C_6H_4$— | 4-F—$C_6H_4$— | 132—133 |
| 132 | 4-$CF_3O$—$C_6H_4$— | 2-MeO—$C_6H_4$— | 115—116 |
| 133 | 2-$CF_2H$—O—$C_6H_4$— | 2-Cl—$C_6H_4$— | 114—115 |
| 134 | 2-$CF_2H$ . $CF_2O$—$C_6H_4$— | 4-F—$C_6H_4$— | 115—116 |
| 135 | 4-$CF_2H$ . $CF_2O$—$C_6H_4$— | 4-F—$C_6H_4$— | 125—126 |
| 136 | 4-$CF_2H$ . $CF_2O$—$C_6H_4$— | 2-F—$C_6H_4$— | 110—111 |
| 137 | 3,4-methylenedioxy-$C_6H_3$— | 4-Cl—$C_6H_4$— | 129—130 |
| 138 | 2-Cl—$C_6H_4$— | 4-EtO—$C_6H_4$— | 94—96 |
| 139 | 2,4-$Cl_2$—$C_6H_3$— | 4-EtO—$C_6H_4$— | 159—161 |
| 140 | 4-Cl—$C_6H_4$— | 4-$NH_2$—$C_6H_4$— | 56 |
| 141 | 4-Me—$C_6H_4$— | 2,4-$Cl_2$—$C_6H_3$— | 183 |
| 142 | 2-Cl—$C_6H_4$— | 2-MeO—$C_6H_4$— | 148—150 |
| 143 | 2-F-4-MeO—$C_6H_3$— | 4-F—$C_6H_4$— | 161—162 |
| 144 | 2-Cl-4-MeO—$C_6H_3$— | 4-F—$C_6H_4$— | 138—140 |
| 145 | 2-Cl-4-MeO—$C_6H_3$— | 4-Cl—$C_6H_4$— | 176—177 |

TABLE I (Cont'd)

| Compound No. | $R^3$ | $R^4$ | Melting point (°C.) |
|---|---|---|---|
| 146 | 2-F-4-MeO—$C_6H_3$— | 2-Cl—$C_6H_4$— | 170—172 |
| 147 | 2-F-4-MeO—$C_6H_3$— | 4-Cl—$C_6H_4$— | 134—136 |
| 148 | 2-Cl-4-Me—$C_6H_3$— | 4-F—$C_6H_4$— | 158 |
| 149 | 4-Cl-2-Me—$C_6H_3$— | 4-F—$C_6H_4$— | 65—67 |
| 150 | 4-Cl-3-Me—$C_6H_3$— | 4-Cl—$C_6H_4$— | 175—176 |
| 151 | cyclobutyl | $C_6H_5$— | oil |
| 152 | 2,4-$Cl_2$—$C_6H_3$— | 2,4-$Cl_2$—$C_6H_3$— | 170 |
| 153 | 3-$NO_2$—$C_6H_4$— | 3-$NO_2$—$C_6H_4$— | 170—172 |

*Includes 1 mole of ethanol occluded in the crystal lattice

+Compounds 8 and 18 were obtained as polymorphs and this explains their different melting points.

**Hydrochloride salt

The composition may be in a conventional pharmaceutical form suitable for oral administration, for example a tablet, a capsule, an emulsion or an aqueous or oily solution or suspension, or suitable for topical application, for example a cream, ointment or gel. The composition may contain conventional pharmaceutical excipients, and may be manufactured by conventional pharmaceutical techniques.

Preferred pharmaceutical or veterinary compositions are compositions suitable for oral administration, and particularly tablets and capsules.

European Patent Application EP—A—0 015 756 referred to above also discloses fungicidal compositions comprising a triazole derivative of the formula I in admixture with an inert carrier or diluent, for example kaolin, bentonite, kieselguhr, dolomite, calcium carbonate, talc, powdered magnesia, Fuller's earth, etc., and optionally including also wetting agents or suspending agents. We have now discovered that a selection of said fungicidal compositions is particularly useful for the treatment of candidosis and human dermatophyte infections.

Thus, according to a further feature of the invention there is provided a composition for use as an antifungal in human or other animals which comprises a triazole derivative of the formula:—

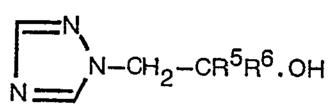

III

wherein $R^5$ and $R^6$, which may be the same or different, are each a phenyl radical bearing a halogen substituent in the 4-position, but excluding the compounds wherein $R^5$ is 2,4-dichlorophenyl or 4-chlorophenyl and $R^6$ is 4-chlorophenyl, or a salt thereof, and an inert carrier or diluent.

It is to be understood that the phenyl radicals $R^5$ and $R^6$ may also bear other substituents, in addition to the halogen substituent in the 4-position, as defined in European Patent Application EP—A—0 015 756, referred to above, and other halogen substituents are preferred.

Suitable halogen substituents, in the 4-position of $R^5$ and $R^6$, or as additional substituents in other positions of $R^5$ and $R^6$ are, for example, chlorine, fluorine and bromine atoms.

Suitable optional other substituents in $R^5$ and $R^6$, in addition to the halogen substituents in the 4-positions, are, for example, nitro, cyano, 1—5C alkyl, 1—4C alkoxy, 1—4C halogenoalkyl and 1—4C halogenoalkoxy, for example methyl, ethyl, propyl (n- or isopropyl), butyl (n-, sec-, iso- and t-butyl), methoxy, ethoxy, propoxy (n- and isopropoxy), butoxy (n-, sec-, iso- and t-butoxy), trifluoromethyl, trichloromethyl, difluoromethyl, dichloromethyl, difluoromethoxy, trifluoromethoxy, 1,1,2,2-tetrafluoroethoxy, pentafluoroethoxy and trifluoroethoxy radicals.

The salts can be salts with inorganic or organic acids, for example hydrochloric, nitric, sulphuric acetic, toluene-p-sulphonic, maleic, tartaric or citric acids.

Examples of particular preferred compounds of the formula III which may be used in the fungicidal composition are those numbered 25, 26, 37, 67, 68, 149, 150 and 152 in Table I.

We have further discovered that, within this class of fungicidal compositions containing as active

ingredient a triazole derivative of the formula II as defined above, and which are particularly useful for the treatment of candidosis and human dermatophyte infections, the selection of the compound 1-(2,4-dichlorophenyl)-1-(4-fluorophenyl)-2-(1,2,4-triazol-1-yl)ethanol confers especially valuable advantages in terms of high *in vivo* fungicidal activity against *Candida* and *Trichophyton* species, and freedom from undesired side effects.

Thus, according to a further feature of the invention there is provided a composition for use as an antifungal in human or other animals, which comprises 1-(2,4-dichlorophenyl)-1-(4-fluorophenyl)-2-(1,2,4-triazol-1-yl)ethanol or a salt thereof, and an inert carrier or diluent.

The antifungal activity of the active ingredients of the compositions of the invention against *Candida albicans,* a causative fungus of candidosis and *Trichlorophyton mentagrophytes,* var. *quinkeanum,* was demonstrated as follows:—

Female mice of around 30 g. weight are injected sub-cutaneously on a Friday with 0.5 mg. of oestradiol benzoate. The following Monday (day 0) they are clipped on the back and then dosed orally with test compounds. They are then inoculated with *Candida albicans* in the vagina and *Trichophyton mentagrophytes* var. *quinkeanum* on the back, and then given a second dose of the same compound. Dosing is repeated once daily on days 1—4. On day 7 skin lesions are scored visually and vaginal samples taken for culture on agar. Groups of 5 mice are used and compounds are dosed initially at a level of 250 mg./kg. The dose is then reduced sequentially until a minimum effective dose (MED) is found. The following table shows minimum effective doses (mg./kg.) giving complete control of infection, for a representative selection of triazole derivatives of the formula II:—

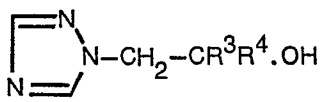 II

| No. in Table I | $R^3$ | $R^4$ | MED v. *C. alibicans* | MED v. *T. mentagrophytes,* var. *quinkeanum* |
|---|---|---|---|---|
| 35 | 4-F-phenyl | 2-F-phenyl | 50 | 50 |
| 33 | 4-Cl-phenyl | 2-F-phenyl | 25 | 25 |
| 34 | 2-Cl-phenyl | 4-F-phenyl | 50 | 25 |
| 25 | 4-Cl-phenyl | 4-F-phenyl | 5 | 5 |
| 26 | 4-F-phenyl | 4-F-phenyl | 5 | 5 |
| 37 | 2,4-diCl-phenyl | 4-F-phenyl | 5 | 5 |

The invention is illustrated but not limited by the following Examples, in which "parts" are by weight:—

Example 1

A mixture of 5, 10, 25, 50, 100 or 250 parts of 1-(2,4-dichlorophenyl)-1-(4-fluorophenyl)-2-(1,2,4-triazol-1-yl)ethanol with 70 parts of calcium carbonate and 200 parts of a 10% maize starch paste is dried and then passed through a 16 mesh screen. 5 parts of magnesium stearate are added and the granules are compressed to give a range of tablets suitable for oral administration for therapeutic purposes.

This active ingredient may be replaced by a therapeutically equivalent amount of any other triazole derivative as hereinbefore defined.

Example 2

A mixture of 2, 5, 10, 25, 50 or 100 parts of 1-(2,4-dichlorophenyl)-1-(4-fluorophenyl)-2-(1,2,4-triazol-1-yl)ethanol, 500 parts of lactose and 100 parts of maize starch is treated with sufficient 10% maize starch paste to give a granular mass. The mixture is passed through a 16-mesh screen, dried, mixed with 8 parts of magnesium stearate and compressed into tablets, giving a range of tablets suitable for oral administration for therapeutic purposes.

The active ingredient may be replaced by a therapeutically equivalent amount of any other triazole derivative as hereinbefore defined.

Example 3

A mixture of 10 parts of 1-(2,4-dichlorophenyl)-1-(4-fluorophenyl)-2-(1,2,4-triazol-1-yl)ethanol

and 190 parts of wheat germ oil is filled into soft gelatin capsules, to give capsules suitable for oral administration for therapeutic purposes.

The active ingredient may be replaced by a therapeutically equivalent amount of any other triazole derivative as hereinbefore defined.

Example 4

A solution of 10 parts of 1-(2,4-dichlorophenyl)-1-(4-fluorophenyl)-2-(1,2,4-triazol-1-yl)ethanol in 83 parts of water, 250 parts of glycerol and 125 parts of ethyl alcohol is mixed with a solution of 300 parts of sucrose in 150 parts of water. A suitable flavouring agent and colouring matter are then added to produce a syrup suitable for oral administration for therapeutic purposes.

The active ingredient may be replaced by a therapeutically equivalent amount of any other triazole derivative as hereinbefore defined.

Example 5

A mixture of 3 parts of gum acacia and 1.5 parts of gum tragacanth is added to a mixture of 1 part of 1-(2,4-dichlorophenyl)-1-(4-fluorophenyl)-2-(1,2,4-triazol-1-yl)ethanol and 33.7 parts of liquid paraffin. To the thoroughly triturated mixture is added slowly with stirring a solution of 0.1 part of cetyl alcoholpolyoxyethylene condensate, 40 parts of sucrose, 0.03 part of propyl p-hydroxybenzoate, 0.3 parts of methyl p-hydroxybenzoate, a suitable flavouring agent and 0.002 part of edible dyestuff in 110 parts of water. The mixture is then homogenized in conventional manner known in the art to produce an emulsion suitable for oral administration for therapeutic purposes.

The active ingredient may be replaced by a therapeutically equivalent amount of any other triazole derivative as hereinbefore defined.

Example 6

A mixture of 0.5 part of finely divided 1-(2,4-dichlorophenyl)-1-(4-fluorophenyl)-2-(1,2,4-triazol-1-yl)ethanol in 3 parts of propylene glycol and 2 parts of ethylene glycol monoethyl ether was added to a stirred mixture of 4 parts of a lanolin and 90.5 parts of molten soft white paraffin. The resulting mixture was allowed to cool to room temperature with rapid stirring, to give a uniform ointment containing 0.5% by weight of active ingredient suitable for topical administration for therapeutic purposes.

The active ingredient may be replaced by another triazole derivative as hereinbefore defined to give similar ointments.

Example 7

A solution was prepared of 1 part of 1-(2,4-dichlorophenyl)-1-(4-fluorophenyl)-2-(1,2,4-triazol-1-yl)ethanol in 20 parts of ethanol and 27 parts of diethylene glycol monethyl ether, then 50 parts of purified water was added, followed by 2 parts of a carboxypolymethylene gelling agent ("Carbapol 940"—trade mark) to give a finely dispersed gel suitable for topical administration for therapeutic purposes.

The active ingredient may be replaced by any other triazole derivative as hereinbefore described.

**Claims**

1. A pharmaceutical or veterinary composition for use as an antifungal in human or other animals, which comprises a triazole derivative of the formula:—

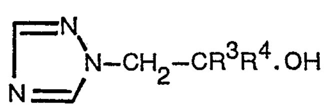 II

wherein $R^3$ is a 1—6C alkyl radical, a 3—7C cycloalkyl radical, a phenyl radical a phenyl radical which is substituted by one or more substituents selected from halogen atoms and 1—5C alkyl, 1—4C alkoxy, 1—4C haloalkyl, 1—4C haloalkoxy, nitro, phenoxy, benzyl, benzyloxy, halobenzyloxy, 1—4C alkylenedioxy, 1—4C haloalkylenedioxy, amino, 1—4C alkylamino, di(1—4C alkyl)amino, hydroxy, morpholino, carboxy and 2—5C alkoxycarbonyl radicals; and $R^4$ is a phenyl, benzyl or $\alpha$-methylbenzyl radical, or a phenyl, benzyl or $\alpha$-methylbenzyl radical each substituted as defined above, or an acid addition salt thereof, but excluding compounds wherein $R^3$ is a 2,4-dichlorophenyl radical and $R^4$ is a phenyl or 2-, 3- or 4-chlorophenyl radical, or $R^3$ is phenyl or 4-chlorophenyl radical and $R^4$ is a 2- or 4-chlorophenyl, together with a pharmaceutically or veterinarily acceptable diluent or carrier.

2. A composition as claimed in claim 1 wherein, in the triazole derivative, $R^3$ is a methyl, ethyl, propyl (n- or iso-propyl), butyl (n-, sec-, iso- or t-butyl), cyclopentyl, cyclohexyl, phenyl or phenyl substituted by one or more substituents selected from fluorine, chlorine and bromine atoms and methyl, ethyl, propyl (n- or isopropyl), butyl (n-, sec-, iso- and t-butyl), methoxy, ethoxy, trifluoromethyl, 1,1,2,2-tetrafluoroethyl, trifluoromethoxy, 1,1,2,2-tetrafluoroethoxy, chlorobenzyloxy, fluorobenzyloxy, methylenedioxy, 1,2-ethylenedioxy, 1,2-propylenedioxy, difluoromethylenedioxy, nitro, amino,

methylamino, dimethylamino, methoxycarbonyl and ethoxycarbonyl radicals, and $R^4$ is a phenyl or benzyl radical each bearing one or more substituents as defined above for a phenyl radical $R^3$, or an acid-addition salt thereof.

3. A composition as claimed in claim 1 wherein, in the triazole derivative, $R^3$ is selected from phenyl, 2-, 3- or 4-chlorophenyl, 2,3- or 2,6-dichlorophenyl, 2-, 3- or 4-fluorophenyl, 2,3- or 2,6-difluorophenyl, 2-, 3- or 4-bromophenyl, 2-chloro-4-fluorophenyl, 2-fluoro-4-chlorophenyl, 2-chloro-6-fluorophenyl, 2-, 3- or 4-methoxyphenyl, 2,4-dimethoxyphenyl, 2-, 3- or 4-ethoxyphenyl, 2-, 3- or 4-nitrophenyl, 2-chloro-4-nitrophenyl, 2-chloro-5-nitrophenyl, 2-, 3- or 4-methylphenyl, 2,4-dimethylphenyl, 2-, 3- or 4-t-butylphenyl, 2-, 3- or 4-trifluoromethylphenyl, 2-, 3- or 4-trifluoromethoxyphenyl, 2-, 3- or 4-(1,1,2,2-tetrafluoroethyl)phenyl, 2,3-(difluoromethylenedioxy)phenyl, 2-fluoro-4-methoxyphenyl, 2-methoxy-4-fluorophenyl, 2-methoxy-4-chlorophenyl, 2-, 3- or 4-phenoxyphenyl, 2-, 3- or 4-phenylphenyl (2-, 3- or 4-biphenylyl), 2-, 3- or 4-benzylphenyl, 2-, 3- or 4-benzyloxyphenyl, 2-, 3- or 4-(4-chloro- or 4-fluoro-benzyloxy)phenyl, 2-, 3- or 4-aminophenyl, 2-, 3- or 4-(N,N-dimethylamino)phenyl, 2-, 3- or 4-(methoxycarbonyl)phenyl and 2-, 3- or 4-morpholinophenyl and $R^4$ is selected from the same radicals, benzyl, $\alpha$-methylbenzyl and the corresponding ring substituted benzyl and $\alpha$-methylbenzyl groups, bearing substituents as defined above in $R^3$.

4. A composition, for use as antifungal in human or other animals, which comprises a triazole derivative of the formula:—

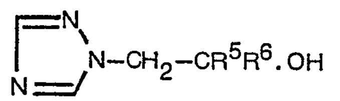 III

wherein $R^5$ and $R^6$, which may be same or different, are each a phenyl radical bearing a halogen substituent in the 4-position, but excluding the compounds wherein $R^5$ is 2,4-dichlorophenyl or 4-chlorophenyl and $R^6$ is 4-chlorophenyl, or a salt thereof, and an inert carrier or diluent.

5. A composition as claimed in claim 4 wherein in the triazole derivative, the halogen substituent is a chlorine, fluorine or bromine atom.

6. A composition as claimed in claim 4 wherein in the triazole derivative, either or both of $R^5$ and $R^6$ bears one or more additional substituents selected from nitro, cyano, 1—5C alkyl, 1—4C alkoxy, 1—4C halogenoalkyl and 1—4 C halogenoalkoxy radicals.

7. A composition adapted for use as an antifungal in human or other animals which comprises 1-(2,4-dichlorophenyl)-1-(4-fluorophenyl)-2-(1,2,4-triazol-1-yl)ethanol or a salt thereof, and an inert carrier or diluent.

8. A composition as claimed in any preceding claim which is in a form suitable for oral administration to mammals.

9. A triazole compound of the formula II as defined in claim 1, or a composition as claimed in claim 1, for use in the treatment of infections of human or other animals caused by micro-organisms.

## Patentansprüche

1. Pharmazeutische oder veterinäre Zusammensetzung für die Verwendung als fungicides Mittel bei Menschen oder Tieren, welche ein Triazolderivat der Formel:

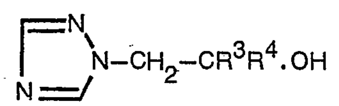 II

worin $R^3$ für ein 1—6C-Alkylradikal, ein 3—7C-Cycloalkylradikal, ein Phenylradikal oder ein durch einen oder mehrere Substituenten substituiertes Phenylradikal steht, wobei die Substituenten ausgewählt sind aus Halogenatomen und 1—5C-Alkyl-, 1—4C-Alkoxy-, 1—4C-Halogenoalkyl-, 1—4C-Halogenoalkoxy-, Nitro-, Phenoxy-, Benzyl-, Benzyloxy-, Halogenobenzyloxy-, 1—4C-Alkylendioxy-, 1—4C-Halogenoalkylendioxy-, Amino-, 1—4C-Alkylamino-, Di(1—4C-alkyl)amino-, Hydroxy-, Morpholino-, Carboxy- und 2—5C-Alkoxycarbonyl-radikalen und $R^4$ für ein Phenyl-, Benzyl- oder $\alpha$-Methyl-benzylradikal oder ein jeweils gemäß obiger Definition substituiertes Phenyl-, Benzyl- oder $\alpha$-Methylbenzylradikal steht, oder ein Säureadditionssalz davon, wobei jedoch Verbindungen ausgeschlossen sind, worin $R^3$ für ein 2,4-Dichlorophenylradikal und $R^4$ für ein Phenyl- oder 2-, 3- oder 4-Chlorophenylradikal steht oder $R^3$ für ein Phenyl- oder 4-Chlorophenylradikal und $R^4$ für ein 2- oder 4-Chlorophenylradikal steht, gemeinsam mit einem pharmazeutisch oder veterinär zulässigen Verdünnungsoder Trägermittel enthält.

2. Zusammensetzung nach Anspruch 1, worin im Triazolderivat $R^3$ für ein Methyl-, Äthyl-, Propyl- (n- oder Isopropyl-), Butyl- (n-, sec-, Iso- oder t-Butyl-), Cyclopentyl-, Cyclohexyl-, Phenyl- oder durch einen oder mehrere Substituenten substituiertes Phenylradikal steht, wobei die Substituenten ausgewählt sind aus Fluor-, Chlor- und Bromatomen und Methyl-, Äthyl-, Propyl- (n- oder Isopropyl-), Butyl- (n-, sec-, Iso- oder t-Butyl-), Methoxy-, Äthoxy-, Trifluoromethyl-, 1,1,2,2-Tetrafluoroäthyl-, Trifluoromethoxy-, 1,1,2,2-Tetrafluoroäthoxy-, Chlorobenzyloxy-, Fluorobenzyloxy-, Methylendioxy-,

1,2-Äthylendioxy-, 1,2-Propylendioxy-, Difluoromethylendioxy-, Nitro-, Amino-, Methylamino-, Dimethylamino-, Methoxycarbonyl- und Äthoxycarbonylradikalen, und $R^4$ für ein Phenyl- oder Benzylradikal steht, das jeweils einen oder mehrere der oben für das Phenylradikal $R^3$ definierte Substituenten trägt, oder worin das Triazolderivat in Form eines Säureadditionssalzes vorliegt.

3. Zusammensetzung nach Anspruch 1, worin im Triazolderivat $R^3$ ausgewählt ist aus Phenyl, 2-, 3- oder 4-Chlorophenyl, 2,3- oder 2,6-Dichlorophenyl, 2-, 3- oder 4-Fluorophenyl, 2,3- oder 2,6-Difluorophenyl, 2-, 3- oder 4-Bromophenyl, 2-Chloro-4-fluorophenyl, 2-Fluoro-4-chlorophenyl, 2-Chloro-6-fluorophenyl, 2-, 3- oder 4-Methoxyphenyl, 2,4-Di-methoxyphenyl, 2-, 3- oder 4-Äthoxyphenyl, 2-, 3- oder 4-Nitrophenyl, 2-Chloro-4-nitrophenyl, 2-Chloro-5-nitrophenyl, 2-, 3- oder 4-Methylphenyl, 2,4-Dimethylphenyl, 2-, 3- oder 4-t-Butylphenyl, 2-, 3- oder 4-Trifluoromethylphenyl, 2-, 3- oder 4-Trifluoromethoxyphenyl, 2-, 3- oder 4-(1,1,2,2-Tetrafluoroäthyl)phenyl, 2,3-(Difluoromethylendioxy)phenyl, 2-Fluoro-4-methoxyphenyl, 2-Methoxy-4-fluorophenyl, 2-Methoxy-4-chlorophenyl, 2-, 3- oder 4-Phenoxyphenyl, 2-, 3- oder 4-Phenylphenyl (2-, 3- oder 4-Biphenylyl), 2-, 3- oder 4-Benzylphenyl, 2-, 3- oder 4-Benzyloxyphenyl, 2-, 3- oder 4-(4-Chloro-oder 4-Fluorobenzyloxy)phenyl, 2-, 3- oder 4-Aminophenyl, 2-, 3- oder 4-(N,N-Dimethylamino)phenyl, 2-, 3- oder 4-(Methoxycarbonyl)phenyl und 2-, 3- oder 4-Morpholinophenyl und $R^4$ ausgewählt ist aus den gleichen Radikalen, Benzyl, $\alpha$-Methylbenzyl und den entsprechenden ringsubstituierten Benzyl- und $\alpha$-Methylbenzylgruppen, die Substituenten tragen, wie sie oben für $R^3$ definiert sind.

4. Zusammensetzung für die Verwendung als fungicides Mittel bei Menschen oder Tieren, welche ein Triazolderivat der Formel:

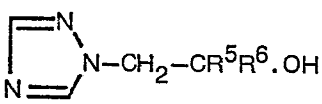

III

worin $R^5$ und $R^6$, welche gleich oder verschieden sein können, jeweils für ein Phenylradikal stehen, das in der 4-Stellung einen Halogensubstituenten trägt, wobei jedoch die Verbindungen ausgeschlossen sind, worin $R^5$ für 2,4-Dichlorophenyl oder 4-Chlorophenyl und $R^6$ für 4-Chlorophenyl steht, oder ein Salz davon sowie ein inertes Träger- oder Verdünnungsmittel enthält.

5. Zusammensetzung nach Anspruch 4, worin im Triazolderivat der Halogensubstituent aus einem Chlor-, Fluor- oder Bromatom besteht.

6. Zusammensetzung nach Anspruch 4, worin im Triazolderivat $R^5$ und/oder $R^6$ einen oder mehrere zusätzliche Substituenten trägt, die ausgewählt sind aus Nitro-, Cyano-, 1—5C-Alkyl-, 1—4C-Alkoxy-, 1—4C-Halogenoalkyl- und 1—4C-Halogenoalkoxyradikalen.

7. Zusammensetzung für die Verwendung als fungicides Mittel bei Menschen oder Tieren, welches 1-(2,4-Dichlorophenyl)-1-(4-fluorophenyl)-2-(1,2,4-triazol-1-yl)-äthanol oder ein Salz davon sowie ein inertes Verdünnungs- oder Trägermittel enthält.

8. Zusammensetzung nach einem der vorhergehenden Ansprüche, welches eine für orale Verabreichung an Säuger geeignete Form aufweist.

9. Die Verwendung einer Triazolverbindung der Formel II, wie sie in Anspruch 1 definiert ist, oder einer Zusammensetzung, wie sie in Anspruch 1 definiert ist, für die Behandlung von durch Mikroorganismen verursachten Infektion bei Menschen oder Tieren.

**Revendications**

1. Composition pharmaceutique ou vétérinaire destinée à être utilisée comme antifongique chez l'homme ou chez d'autres animaux, qui comprend un dérivé de triazole de formule:

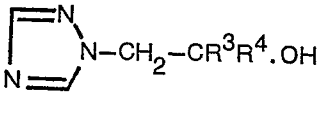

II

dans laquelle $R^3$ est un radical alkyle en $C_1$ à $C_6$, un radical cycloalkyle en $C_3$ à $C_7$, un radical phényle, un radical phényle qui porte un ou plusieurs substituants choisis entre des atomes d'halogènes et des radicaux alkyle en $C_1$ à $C_5$, alkoxy en $C_1$ à $C_4$, halogénalkyle en $C_1$ à $C_4$, halogénalkoxy en $C_1$ à $C_4$, nitro, phénoxy, benzyle, benzyloxy, halogénobenzyloxy, alkylènedioxy en $C_1$ à $C_4$, halogénalkylènedioxy en $C_1$ à $C_4$, amino, alkylamino en $C_1$ à $C_4$, di-(alkyle en $C_1$ à $C_4$)-amino, hydroxy, morpholino, carboxy et alkoxycarbonyle en $C_2$ à $C_5$; et $R^4$ est un radical phényle, benzyle ou $\alpha$-méthylbenzyle, ou un radical phényle, benzyle ou $\alpha$-méthylbenzyle dont chacun est substitué comme défini ci-dessus, ou un sel d'addition d'acide de ce dérivé, mais à l'exclusion des composés dans lesquels $R^3$ est un radical 2,4-dichlorophényle et $R^4$ est un radical phényle ou un radical 2-, 3- ou 4-chlorophényle, ou bien $R^3$ est un radical phényle ou 4-chlorophényle et $R^4$ est un radical 2- ou 4-chlorophényle, conjointement avec un diluant ou support acceptable du point de vue pharmaceutique ou vétérinaire

2. Composition suivant la revendication 1 pour laquelle, dans le dérivé de triazole, $R^3$ est un radical méthyle, éthyle, propyle (n- ou isopropyle), butyle (n-, sec.-, iso- ou tertio-butyle), cyclopentyle, cyclohexyle, phényle ou phényle substitué avec un ou plusieurs substituants choisis entre des atomes

de fluor, chlore et brome et des substituants méthyle, éthyle, propyle (n- ou isopropyle), butyle (n-, sec.-, iso- et tertio-butyle), méthoxy, éthoxy, trifluorométhyle, 1,1,2,2-tétrafluoréthyle, trifluorométhoxy, 1,1,2,2-tétrafluoréthoxy, chlorobenzyloxy, fluorobenzyloxy, méthylènedioxy, 1,2-éthylènedioxy, 1,2-propylènedioxy, difluorométhylènedioxy, nitro, amino, méthylamino, diméthylamino, méthoxycarbonyle et éthoxycarbonyle, et $R^4$ est un radical phényle ou benzyle, chacun portant un ou plusieurs substituants, comme défini ci-dessus, pour un radical phényle $R^3$, ou un sel d'addition d'acide de ce dérivé.

3. Composition suivant la revendication 1, pour laquelle, dans le dérivé de triazole, $R^3$ est choisi entre des radicaux phényle, 2-, 3- ou 4-chlorophényle, 2,3- ou 2,6-dichlorophényle, 2-, 3- ou 4-fluorophényle, 2,3- ou 2,6-difluorophényle, 2-, 3- ou 4-bromophényle, 2-chloro-4-fluorophényle, 2-fluoro-4-chlorophényle, 2-chloro-6-fluorophényle, 2-, 3- ou 4-méthoxyphényle, 2,4-diméthoxyphényle, 2-, 3- ou 4-éthoxyphényle, 2-, 3- ou 4-nitrophényle, 2-chloro-4-nitrophényle, 2-chloro-5-nitrophényle, 2-, 3- ou 4-méthylphényle, 2,4-diméthylphényle, 2-, 3- ou 4-tertio-butylphényle, 2-, 3- ou 4-trifluorométhylphényle, 2-, 3- ou 4-trifluorométhoxyphényle, 2-, 3- ou 4-(1,1,2,2-tétrafluoréthyl)phényle, 2,3-(difluorométhylènedioxy)phényle, 2-fluoro-4-méthoxyphényle, 2-méthoxy-4-fluorophényle, 2-méthoxy-4-chlorophényle, 2-, 3- ou 4-phénoxyphényle, 2-, 3- ou 4-phénylphényle (2-, 3- ou 4-biphénylyle), 2-, 3- ou 4-benzylphényle, 2-, 3- ou 4-benzyloxyphényle, 2-, 3- ou 4-(4-chloro- ou 4-fluorobenzyloxy)phényle, 2-, 3- ou 4-aminophényle, 2-, 3- ou 4-(N,N-diméthylamino)phényle, 2-, 3- ou 4-(méthoxycarbonyl)phényle et 2-, 3- ou 4-morpholinophényle, et $R^4$ est choisi entre les mêmes radicaux, les groupes benzyle, $\alpha$-méthylbenzyle et les groupes benzyle et $\alpha$-méthylbenzyle correspondants substitués sur le noyau, portant des substituants tels que définis ci-dessus pour $R^3$.

4. Composition destinée à être utilisée comme antifongique chez l'homme ou d'autres animaux, qui comprend un dérivé de triazole de formule:

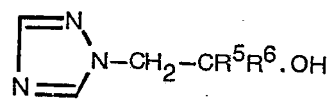

III

dans laquelle $R^5$ et $R^6$, qui peuvent être égaux ou différents, représentent chacun un radical phényle portant un substituant halogéno ou 4-chlorophényle en position 4, mais à l'exclusion des composés dans lesquels $R^5$ est un radical 2,4-dichlorophényle et $R^6$ est un radical 4-chlorophényle, ou un sel de ce dérivé, et un support ou diluant inerte.

5. Composition suivant la revendication 4, pour laquelle, dans le dérivé de triazole, le substituant halogéno est un atome de chlore, de fluor ou de brome.

6. Composition suivant la revendication 4, pour laquelle, dans le dérivé de triazole, l'un ou l'autre de $R^5$ et $R^6$ ou les deux portent un ou plusieurs autres substituants choisis entre des radicaux nitro, cyano, alkyle en $C_1$ à $C_5$, alkoxy en $C_1$ à $C_4$, halogénalkyle en $C_1$ à $C_4$ et halogénalkoxy en $C_1$ à $C_4$.

7. Composition destinée à être utilisée comme antifongique chez l'homme ou d'autres animaux, qui comprend du 1-(2,4-dichlorophényl)-1-(4-fluorophényl)-2-(1,2,4-triazole-1-yl)éthanol ou un sel de ce composé, et un support ou diluant inerte.

8. Composition suivant l'une quelconque des revendications précédentes, qui est sous une forme propre à l'administration orale à des mammifères.

9. Composé de triazole de formule II tel que défini dans la revendication 1, ou composition telle que définie dans la revendication 1, destiné au traitement d'infections dues à des micro-organismes chez l'homme ou d'autres animaux.